# EUROPEAN PATENT SPECIFICATION

(11) **EP 3 833 248 B1**
(45) Date of publication and mention of the grant of the patent: **23.10.2024**
(21) Application number: 19769897.0
(22) Date of filing: 07.08.2019
(51) Int. Cl.: A61B 5/0205, A61B 5/0295, A61B 5/053, A61B 5/00, A61B 5/318

(54) **METHOD AND BIOMEDICAL ELECTRONIC EQUIPMENT FOR MONITORING PATIENT'S CONDITION AFTER A STROKE**
VERFAHREN UND BIOMEDIZINISCHE ELEKTRONISCHE AUSRÜSTUNG ZUR ÜBERWACHUNG DES ZUSTANDS EINES PATIENTEN NACH EINEM SCHLAGANFALL
PROCÉDÉ ET ÉQUIPEMENT ÉLECTRONIQUE BIOMÉDICAL POUR SURVEILLER L'ÉTAT DE PATIENT APRÈS UN ACCIDENT VASCULAIRE CÉRÉBRAL

(30) Priority: 08.08.2018 LT 2018537
(43) Date of publication of application: 16.06.2021
(73) Proprietor: Kaunas University of Technology, 44249 Kaunas (LT); Lietuvos sveikatos mokslu universitetas, 44307 Kaunas (LT); UAB Gruppo Fos Lithuania, 09303 Vilnius (LT)
(72) Inventor: JEGELEVICIUS, Darius, Kaunas (LT); LUKOSEVICIUS, Arunas, Kaunas (LT); MAROZAS, Vaidotas, Kaunas (LT); PETRENAS, Andrius, Kaunas (LT); DAUKANTAS, Saulius, Kaunas (LT); SIMAITYTE, Monika, Kaunas (LT); RASTENYTE, Daiva, 44307 Kaunas (LT); MATIJOSAITIS, Vaidas, 44307 Kaunas (LT); LAUCKAITE, Kristina, 44307 Kaunas (LT); MAKAUSKIENE, Rosita, 01301 Vilnius (LT); MIKULENAS, Mantas, 51423 Kaunas (LT); BOERO, Federico, 51423 Kaunas (LT); SANSALONE, Andrea, 51423 Kaunas (LT)
(74) Representative: Klimaitiene, Otilija
(86) International application number: PCT/IB2019/056718
(87) International publication number: WO 2020/031104

(56) References cited:
- EP-A1- 3 031 395
- JUAN J PEREZ: "To what extent is the bipolar rheoencephalographic signal contaminated by scalp blood flow? A clinical study to quantify its extra and non-extracranial components", BIOMEDICAL ENGINEERING ONLINE, BIOMED CENTRAL LTD, LONDON, GB, vol. 13, no. 1, 6 September 2014 (2014-09-06), pages 131, XP021197341, ISSN: 1475-925X, DOI: 10.1186/1475-925X-13-131

## Description

### FIELD OF INVENTION

This invention relates to medical equipment in general and more particularly to the equipment and measurement-analysis method for monitoring stroke-induced brain and cardiovascular functional parameters and their dynamics after the stroke, by employing bioimpedance, electrocardiographic, photoplethysmographic and motion analysis.

### BACKGROUND ART

This disclosure provides a method and its hardware implementation for monitoring stroke-induced brain and cardiovascular functional parameters and their dynamics after the stroke. The invention is novel in that the post-stroke state is evaluated non-invasively by measuring two sets of parameters. The first set comprises multi-channel parameters derived from the measurement of electrical bio-impedance of the brain. The second set comprises multi-channel cardiovascular system parameters related to post-stroke condition, which are obtained from electrocardiography, plethysmography, and human motion sensors. All measurements are performed continuously and it is synchronised with the electrocardiogram. The time-varying bio-impedance parameters measured in many measurement channels are used to determine changes in brain tissue and their spatial distribution. Measurement data from different (multimodal) sources are combined and analyzed in a complex way, thus providing more information than analyzing each of the different measurements separately, in a non-synchronized manner.

Patent US8211031B2 (published July 3, 2012) discloses a device and method for measurement of physiological parameters by using the electrical impedance of brain tissue. The measurement of electrical impedance alone does not provide an evaluation of stroke-induced changes in parameters of cardiovascular system, as well as possibility to synchronize different types of measurement. There is also no information about spatial analysis of recorded data.

Patent application US20110190600A1 (published August 4, 2011) describes a system of physiological sensors and a measurement method by using these sensors. The document lists different sensors (biopotential electrodes, optical detectors, temperature sensors, etc.) and provides a measurement method for those sensors. However, it does not mention the processing, synchronization and analysis algorithm of the recorded signals. It remains unclear how these sensors can be linked to the system to achieve a specific study result, since the principles of linking the data recorded by different sensors are not presented.

The above presented technical solutions have the following disadvantages:
- electrical impedance or other parameters of brain tissue are measured separately and are not used in combination to provide new information. Parameters of brain tissue are not in combination with other parameters of cardiovascular system, i.e., there is a lack of versatile methods for data recording and holistic analysis;
- electrical measurement alone does not allow to measure parameters associated with condition-induced blood pressure;
- a system with several physiological sensors is presented, however, no integration and computation is included to provide quantitative parameters for evaluation patient's condition after a stroke;
- there is no possibility to monitor the spatial distribution of the brain tissue changes over time
European patent application No. EP3031395 discloses a method of evaluating patients suspected of suffering from an acute stroke, the method comprising: a) obtaining signals of impedance plethysmography (IPG), photoplethysmography (PPG) or both, in the patient; b) processing the one or more signals to obtain one or more measures of cerebral hemodynamics comprising an estimate of cerebral blood flow, of the patient; and c) notifying medical personnel if the estimate of cerebral blood flow increases by a predetermined relative or absolute amount. Main disadvantages of the method is that it does not provide means for: 1) bioimpedance measurements in different electric current frequencies; 2) increasing signal-to-noise ratio of bioimpedance measurements; 3) acquisition of bioimpedance data in a way suitable for tomographic reconstruction.

Our suggested invention provides a new technical solution without the above-listed drawbacks.

### SUMMARY OF INVENTION

The present invention provides a novel method and equipment for non-invasively monitoring and analysis of post-stroke changes in the human brain and recognizing functional pathologies of the cardiovascular system (e.g., paroxysmal atrial fibrillation, hypertension) associated with brain stroke. It presents a portable non-invasive device with the implemented measurement-analytical method that enables monitoring of heart function and selectively of brain tissue functions and their changes by measuring electrical bioimpedance along with electrocardiogram (ECG) and photoplethysmogram (PPG) and patient's motion measurements.

The presented method and equipment allow to measure two types of parameters:
- electrical impedance of the brain and cardiovascular system parameters, as well as to process, synchronize, analyze them and thus to obtain information about the condition of cardio and brain vascular activity changes over time.
- also, spatial analysis is applied to the measured bioimpedance data which together with the temporal analysis allows to determine the patient's condition after stroke and its changes over time.

### DESCRIPTION OF DRAWINGS

FIG. 1 is a schematic diagram of the cardiovascular monitoring (cardio-plethysmographic) equipment.
FIG. 2 is a schematic diagram of the equipment for monitoring and evaluating cerebral blood flow and cerebral conduction (by measurement of head electrical bioimpedance).

### DRAWINGS - Reference Numerals

1 - part of the cardiovascular monitoring (cardio - plethysmographic) equipment,
1.1 - sensors
1.1.1 - ECG electrodes
1.1.2 - PPG sensors
1.1.3 - inertial measurement unit (IMU)
1.1.4 - altimeter
1.2 - data recording
1.2.1 - ECG recording
1.2.2 - PPG recording
1.2.3 - recording of motion data
1.2.4 - array of synchronized data
1.3 - data processing algorithms
1.3.1 - recognition and characterization of atrial fibrillation arrhythmias
1.3.2 - determination of pulse wave arrival time
1.3.3 - indirect blood pressure measurements
1.4 - data output, characterization
2 - part of the equipment for monitoring and evaluating the cerebral blood flow and cerebral electrical conductivity (by measuring electrical bioimpedance of the brain)
2.1 - sensors
2.1.1 - bioimpedance sensors
2.1.3 - scalp blood circulation sensor and cuff
2.2 - data gathering
2.2.1 - interconnection of electrodes (multiplexing)
2.2.2 - data aquisition and processing
2.2.3 - voltage sensing
2.2.4 - creating and supplying electrical current
2.2.5 - control device
2.2.6 - synchronized data array
2.3 - data processing algorithms
2.3.1 - analysis of temporal parameters
2.3.2 - analysis of spatial parameters

The presented figures are illustrative only, the scale, proportions and other aspects do not necessarily correspond to the actual technical solution.

### DETAILED DESCRIPTION OF PREFERRED EMBODIMENTS

This description provides a method and equipment for monitoring the condition of a person who has suffered a stroke and for predicting potential risks. A new technical solution can be used to detect neurophysiological changes, to detect atrial fibrillation, increased variability of blood pressure parameters, and to assess the risk of the secondary stroke. The equipment can be used both for continuous monitoring and for episodic monitoring. The equipment can be used in hospitals or other medical facilities, as well as in patient's home, or other similar environments.

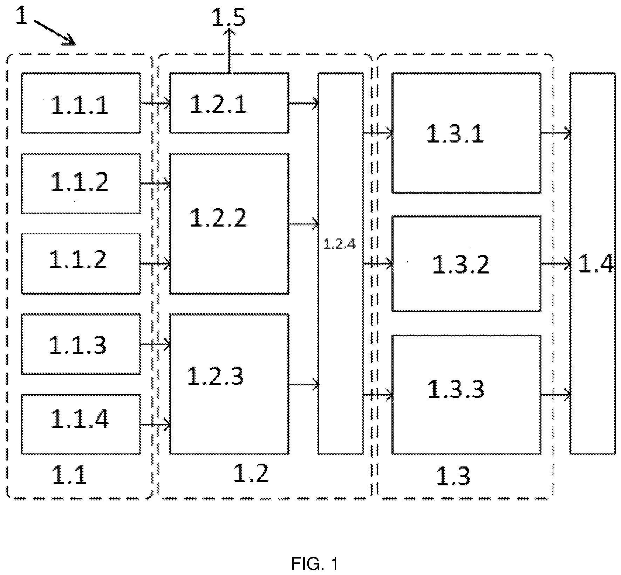

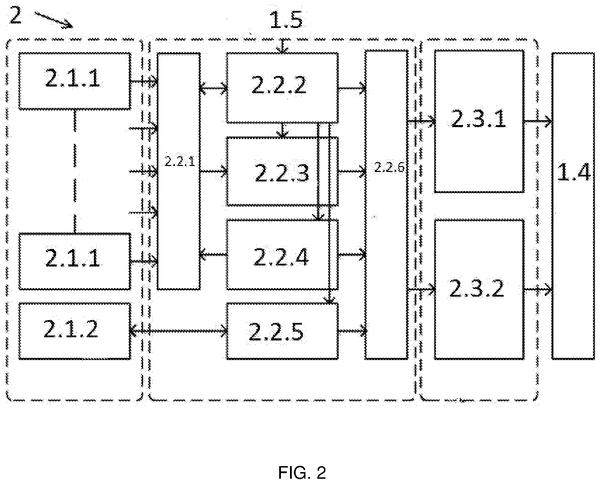

This technical solution and the equipment consists of least these two components or functional units:
- part of the equipment for cardiovascular monitoring (cardio-plethysmographic) (1), (Fig. 1);
- part of equipment for monitoring and evaluation of cerebral blood flow and cerebral electrical conductivity (by measuring of electrical impedance of the brain) (2) (Fig. 2)

The bioimpedance part (2) is used to measure the electrical resistance of the scalp and brain tissues. Such equipment has bioimpedance sensors (2.1.1) which are positioned closely onto the head. One pair of electrodes emits an electrical signal of a certain frequency and intensity which are generated in the current supplying device (2.2.4), and the other pair of electrodes receives the signal and transmits it to a the voltage measurement device (2.2.3) where the impedance of the scalp and brain tissues is calculated. Several pairs of such electrodes may be used. In the present invention, not only the temporal characteristics of electrical signals (2.3.1), synchronized with the cardiac cycles seen in the electorcardiogram (1.1), are measured and analyzed, but also the spatial characteristics of brain tissues (2.3.2). In this case, the spatial characteristics mean that the measurements are intended to determine the impedance distribution in the brain tissue.
According to the present invention, the bioimpedance part (2) has at least the following basic functional components: sensors (2.1), data reception (2.2.2), data processing (2.2.2) and data output, visualization (1.4). One pair of electrodes delivers a predefined frequency alternating current signal that penetrates through the head tissue and creates a potential difference recorded by another pair of electrodes. The recorded signal is transmitted to the functional part of the data gathering (2.2), where the electrical signals are processed, synchronized with (1.1.1) the ECG sensor signal, thus forming a data array (1.2.4) which is transmitted to the functional part of data analysis (2.3). In the data processing functional part (2.3), array of the recorded data (1.2.4) are analyzed to determine the temporal and spatial distribution characteristics of the bioimpedance. The result of the array (1.2.4) processing is represented by the functional part of the result visualization (1.4).

To collect signals, the device employs several methodologies and combines adjacent and opposite methodologies, by using their opposite properties to estimate potential difference at the surface and depths of the brain tissue. The device also can capture signals using techniques of intersecting and all possible positioning, avoiding repetitive or reversed polarity measurements.

The device reduces the effects of scalp blood flow noise by using a cuff to block the blood flow to the scalp before measurement, and a photoplethysmographic sensor to monitor the success of the blood flow blocking, which gives a signal that the cuff is already tightened.

The device for tomographic measurements of brain impedance can use a different frequency of the supplied current to control the current path at the cellular level of head tissues.

The device can perform reoencephalography measurements of temporal changes in cerebral blood flow, along with impedance, ECG and PPG measurements.

Signal strength is controlled by passive and active methods. The signal is amplified in the analog-to-digital converter (ADC), thus increasing the power of the useful signal. The signal is also amplified by increasing the current amplitude to a maximum permissible level depending on the frequency (10mA at 100kHz).

For impedance scanning, the device uses 24-bit ADC increasing the dynamic range of measurements.

The device has two configurations of 16 and 32 channels providing different measurement accuracy.

As mentioned above, the bioimpedance system (2) measures the impedance of head tissues, therefore, the implementation of this signal measurement system part has to be adapted to fit it onto the head. The shape may resemble a hat, helmet or similar.

The cardiovascular monitoring part (1) is designated to assess the patient's condition after a stroke because this part (1) is related to cerebral blood circulation. Cardiovascular monitoring (1) allows to measure parameters such as atrial fibrillation, pulse rate, and blood pressure, which are important for assessing the patient's condition after a stroke. The recorded electrocardiogram is also used to synchronize data between the two parts of the system (1.1). The cardiovascular monitoring portion (1) can be implemented as a wearable wristband.

The cardiovascular monitoring part (1) consists of at least the following basic functional components: sensors (1.1), data acquisition components (1.2), data processing (1.2), and result visualization tools (1.4). The functional part of the sensors consists of several types of sensors: electrocardiogram (ECG) sensors (1.1.1), at least two photoplethysmography (PPG) sensors (1.1.2), inertial motion sensors (1.1.3) (accelerometers, gyroscopes), altimeter (height sensor) (1.1.4). The recorded signals by all sensors are transmitted to the functional part of data receiving (1.2.1), where the signals are filtered, amplified, digitized, synchronized to the electrocardiogram signal (1.5) and further transmitted to the functional part of data processing (1.3). In the functional part of data processing (1.3), data is processed to detect atrial fibrillation (1.3.1), measure pulse wave arrival (1.3.2) and indirectly evaluate changes in blood pressure (1.3.3). The result of data processing is represented by the functional part of the result representation (1.4), which can also be used to represent bioimpedance data.

The method implemented in the equipment comprises at least the following operating steps:
- measuring the electric bioimpedance of the head comprising data set Z(x,y,t,f), in the measurement part (2);
- measuring the data for cardio-plethysmographic dataset ECG-PPG(t), in cardio-plethysmographic part (1);
- synchronization of impedance data cardio-plethysmographic data set with an electrocardiogram (1.5) to obtain an array of synchronized data (1.2.4);
- applying adaptive data processing and analysis algorithms for the synchronized data array (1.2.4), such as:
   ∘ truncated Landweber iteration scheme is applied for the evaluation of spatial distribution of the bioimpedance;
   ∘ the variability of blood pressure parameters is evaluated by the variability of pulse wave arrival time measured by using the wristband;
   ∘ detection of atrial fibrillation episodes is based on the evaluation of pulse irregularity;
   ∘ the concentration of arrhythmia episodes is evaluated by the episode aggregation or density parameter.
- present the processed and analyzed data by the data display means (1.4) and, if necessary, provide for further processing or presentation.

The above computational steps are implemented by electronic computing means connected to the equipment parts that measure the above listed physical parameters, as well as connected by communication means to remote electronic computing devices. The aforementioned electronic computing means are:
- electronic devices with a processor (processors) for processing data,
- cache and/or permanent storage devices for storing data and information,
- internal communication means for communication between components,
- communication devices with external devices.

Aforementioned means may be realized as a computer or a microcontroller with a special software implementing the above-listed computation steps. The electronic equipment implementing analysis and computation may be located at a distance from the measuring equipment. The aforementioned equipment may be portable.

To illustrate and describe the present invention, the following description of the preferred embodiments is provided above. It is not a detailed or restrictive description to determine the exact form or embodiment. The description above should be viewed as an illustration rather than a limitation. Many modifications and variations may be apparent to those skilled in the art. For those skilled in the art, an embodiment is selected and described in order to allow to understand the principles of the invention and its best practical application for different embodiments with different modifications suitable for a particular application or implementation application. The scope of the invention is defined by the claims, in which all the foregoing terms have the widest possible meaning unless otherwise stated.

Embodiments described by those skilled in the art can be made with amendments and modifications, without departing from the scope of the present invention as defined by the claims of the invention.

## Claims

1. A method for monitoring patient's condition after a stroke, comprising:
- recording head's electrical bioimpedance data (2) to form an impedance data set Z(x, y, t, f);
- recording cardio-plethysmographic data (1) comprising ECG(t) and FPG(t) cardio-plethysmographic data sets;
- the impedance and cardio-plethysmographic data sets are synchronized according to an electrocardiogram (1.5), resulting in an array of synchronized data (1.2.4), where processing and analysis algorithms are applied to the array of synchronized data (1.2.4)
- applying data processing and analysis algorithms to an array of data,
- presenting the processed and analyzed data by data display means (1.4), and if necessary, transmitting the presented processed and analyzed data for further processing or presentation.
**characterized in that**
- spatial distribution of brain parameters is analyzed for determining temporal and spatial distribution characteristics of the bioimpedance by using an electric current of different frequencies, where
- strength of the impedance measurement signal is controlled by a supply current depending on the frequency of the supplied current and amplification of the signal.

2. The method for monitoring patient's condition after a stroke according to claim 1, wherein the measured data (2) of the electrical bioimpedance of the brain is processed to locate stroke-induced changes in brain tissue, its functional changes, as well as record their association with changes of the cardiovascular system.

3. The method for monitoring patient's condition after a stroke according to claims 1 to 2, wherein at least one algorithm of estimating the blood pressure variability associated with stroke is based on the simultaneous use of electrocardiographic and photoplethysmographic signals, recorded on a wristband.

4. The method for monitoring patient's condition after a stroke according to claims 1 to 3, wherein the impedance signals are collected by using a combination of opposite (with current electrodes facing each other) and adjacent (with current electrodes being adjacent) measurements.

5. The method for monitoring patient's condition after a stroke according to claims 1 to 4, wherein influence of scalp blood flow noise on impedance signals is reduced by blocking the scalp blood flow prior to measurement, by using an inflatable cuff with a built-in photoplethysmographic sensor for tracking the blood flow and giving a signal that the cuff was tightened enough.

6. The method for monitoring patient's condition after a stroke according to claims 1 to 5, wherein reoencephalography measurements of the cerebral blood flow temporal changes are performed along with impedance and ECG and PPG.

## Patentansprüche

1. Verfahren zum Überwachen eines Zustands eines Patienten nach einem Schlaganfall, das umfasst:
- Aufzeichnen von elektrischen Bioimpedanzdaten (2) des Kopfes, um einen Impedanzdatensatz Z(x, y, t, f) zu bilden;
- Aufzeichnen von kardioplethysmographischen Daten (1), die kardioplethysmographische EKG(t)- und FPG(t)-Datensätze umfassen;
- die Impedanz- und kardioplethysmographischen Datensätze gemäß einem Elektrokardiogramm (1.5) synchronisiert werden, wobei ein Feld von synchronisierten Daten (1.2.4) erhalten wird, wobei Verarbeitungs- und Analysealgorithmen auf das Feld von synchronisierten Daten (1.2.4) angewandt werden,
- Anwenden von Datenverarbeitungs- und Datenanalysealgorithmen auf ein Datenfeld,
- Präsentieren der verarbeiteten und analysierten Daten mithilfe eines Datenanzeigemittels (1.4) und bei Bedarf Übertragen der präsentierten verarbeiteten und analysierten Daten für eine weitere Verarbeitung oder Präsentation.
**dadurch gekennzeichnet, dass**
- eine räumliche Verteilung von Hirnparametern zum Bestimmen von Charakteristika einer zeitlichen und räumlichen Verteilung der Bioimpedanz unter Verwendung eines elektrischen Stroms verschiedener Frequenzen analysiert wird, wobei
- eine Stärke des Impedanzmesssignals durch einen Speisestrom je nach Frequenz des gespeisten Stroms und Verstärkung des Signals gesteuert wird.

2. Verfahren zum Überwachen eines Zustands eines Patienten nach einem Schlaganfall nach Anspruch 1, wobei die gemessenen Daten (2) der elektrischen Bioimpedanz des Gehirns verarbeitet werden, um schlaganfallinduzierte Veränderungen in Hirngewebe bzw. dessen funktionelle Veränderungen zu lokalisieren sowie deren Zusammenhang mit Veränderungen des kardiovaskulären Systems aufzuzeichnen.

3. Verfahren zum Überwachen eines Zustands eines Patienten nach einem Schlaganfall nach den Ansprüchen 1 bis 2, wobei zumindest ein Algorithmus zum Schätzen der Blutdruckvariabilität in Zusammenhang mit einem Schlaganfall auf der gleichzeitigen Verwendung von elektrokardiographischen und photoplethysmographischen Signalen basiert, die auf einem Armband aufgezeichnet werden.

4. Verfahren zum Überwachen eines Zustands eines Patienten nach einem Schlaganfall nach den Ansprüchen 1 bis 3, wobei die Impedanzsignale unter Verwendung einer Kombination von gegenüberliegenden (wobei Stromelektroden einander zugewandt sind) und benachbarten (wobei Stromelektroden einander benachbart liegen) Messungen gesammelt werden.

5. Verfahren zum Überwachen eines Zustands eines Patienten nach einem Schlaganfall nach den Ansprüchen 1 bis 4, wobei ein Einfluss eines Kopfhautdurchblutungsgeräuschs auf Impedanzsignale durch Blockieren der Kopfhautdurchblutung vor einer Messung unter Verwendung einer aufblasbaren Manschette mit einem integrierten photoplethysmographischen Sensor zum Verfolgen des Blutflusses und Erzeugen eines Signals, dass die Manschette ausreichend zusammengezogen wurde, verringert wird.

6. Verfahren zum Überwachen eines Zustands eines Patienten nach einem Schlaganfall nach den Ansprüchen 1 bis 5, wobei reoenzephalographische Messungen von zeitlichen Veränderungen der Hirndurchblutung gemeinsam mit Impedanz und EKG und PPG durchgeführt werden.

## Revendications

1. Procédé de surveillance de l'état d'un patient après un accident vasculaire cérébral, comprenant :
- l'enregistrement de données de bioimpédance électrique de la tête (2) pour former un ensemble de données d'impédance Z(x, y, t, f) ;
- l'enregistrement de données cardio-pléthysmographiques (1) comprenant des ensembles de données cardio-pléthysmographiques ECG(t) et FPG(t) ;
- les ensembles de données d'impédance et cardio-pléthysmographiques sont synchronisés selon un électrocardiogramme (1.5), ce qui donne un tableau de données synchronisées (1.2.4), où des algorithmes de traitement et d'analyse sont appliqués au tableau de données synchronisées (1.2.4)
- l'application d'algorithmes de traitement et d'analyse de données à un tableau de données,
- la présentation des données traitées et analysées par un moyen d'affichage de données (1.4), et si nécessaire, la transmission des données traitées et analysées présentées pour un traitement ou une présentation ultérieur.
**caractérisé en ce que**
- la distribution spatiale des paramètres cérébraux est analysée pour déterminer les caractéristiques de distribution temporelle et spatiale de la bioimpédance à l'aide d'un courant électrique de différentes fréquences, où
- l'intensité du signal de mesure d'impédance est commandée par un courant d'alimentation dépendant de la fréquence du courant fourni et de l'amplification du signal.

2. Procédé de surveillance de l'état d'un patient après un accident vasculaire cérébral selon la revendication 1, dans lequel les données mesurées (2) de la bioimpédance électrique du cerveau sont traitées pour localiser des changements induits par l'accident vasculaire cérébral dans le tissu cérébral, ses changements fonctionnels, ainsi que pour enregistrer leur association avec des changements du système cardiovasculaire.

3. Procédé de surveillance de l'état d'un patient après un accident vasculaire cérébral selon les revendications 1 à 2, dans lequel au moins un algorithme d'estimation de la variabilité de la pression artérielle associée à l'accident vasculaire cérébral est basé sur l'utilisation simultanée de signaux électrocardiographiques et photopléthysmographiques, enregistrés sur un bracelet.

4. Procédé de surveillance de l'état d'un patient après un accident vasculaire cérébral selon les revendications 1 à 3, dans lequel les signaux d'impédance sont collectés à l'aide d'une combinaison de mesures opposées (avec des électrodes de courant face à face) et adjacentes (avec des électrodes de courant adjacentes).

5. Procédé de surveillance de l'état d'un patient après un accident vasculaire cérébral selon les revendications 1 à 4, dans lequel l'influence du bruit du flux sanguin du cuir chevelu sur les signaux d'impédance est réduite en bloquant le flux sanguin du cuir chevelu avant la mesure, à l'aide d'un brassard gonflable muni d'un capteur photopléthysmographique intégré pour suivre le flux sanguin et donner un signal indiquant que le brassard a été suffisamment serré.

6. Procédé de surveillance de l'état d'un patient après un accident vasculaire cérébral selon les revendications 1 à 5, dans lequel des mesures de rhéoencéphalographie des variations temporelles du flux sanguin cérébral sont effectuées avec l'impédance, l'ECG et le PPG.
